# EUROPÄISCHE PATENTSCHRIFT

(11) **EP 1 481 700 B1**
(45) Veröffentlichungstag und Bekanntmachung des Hinweises auf die Patenterteilung: **22.01.2014**
(21) Anmeldenummer: 04012654.2
(22) Anmeldetag: 27.05.2004
(51) Int. Cl.: A61M 1/36, A61M 1/34

(54) **Vorrichtung zur extrakorporalen Bestrahlung einer Bilirubin enthaltenden Flüssigkeit**
Device for extracorporeal irradiation of a liquid containing bilirubin
Dispositif pour l'irradiation extracorporelle d'un liquide contenant de la bilirubine

(30) Priorität: 30.05.2003 DE 10324668
(43) Veröffentlichungstag der Anmeldung: 01.12.2004
(73) Patentinhaber: Fresenius Medical Care Deutschland GmbH, 61352 Bad Homburg (DE)
(72) Erfinder: Wüpper, Andreas, Dr., 60437 Frankfurt (DE)
(74) Vertreter: Müller-Boré & Partner Patentanwälte PartG mbB

(56) Entgegenhaltungen:
- WO-A-01/94349
- DE-A- 19 914 850
- US-A- 5 744 042
- TOTSUNE K: "INNOVATION IN BLOOD PURIFICATION FOR HEPATIC FAILURE DIRECT PHOTOIRRADIATION OF PLASMA" ASAIO TRANSACTIONS, HARPER AND ROW PUBLISHERS, HAGERSTOWN, MD, US, Bd. 32, Nr. 1, 1. Juli 1986 (1986-07-01), Seiten 138-142, XP002026281
- PATENT ABSTRACTS OF JAPAN Bd. 0130, Nr. 95 (C-573), 6. März 1989 (1989-03-06) & JP 63 275351 A (KURARAY CO LTD), 14. November 1988 (1988-11-14)

## Beschreibung

Die vorliegende Erfindung betrifft eine Vorrichtung zur extrakorporalen Bestrahlung von einer Bilirubin enthaltenden Flüssigkeit, wobei Bilirubin in seine wasserlöslichen Photoisomere überführt wird. Des weiteren wird ein Verfahren zur Reduzierung des Bilirubinspiegels in einem Menschen beschrieben.

Bilirubin entsteht beim Abbau von Hämoglobin und wird im Menschen in einer Rate von ungefähr 300 mg/Tag gebildet. Es ist praktisch nicht wasserlöslich und kann daher nicht über die Niere eliminiert werden. Bilirubin bindet an Albumin und wird so im Blut transportiert (sogenanntes unkonjugiertes oder indirektes Bilirubin). In der Leber wird Bilirubin durch Reaktion mit Glucuronsäure in ein wasserlösliches Produkt (sogenanntes konjugiertes oder direktes Bilirubin) überführt. Dieses konjugierte Bilirubin wird mit der Galle in den Darm ausgeschieden, wo es durch Bakterien zu einer Reihe von verschiedenen Produkten, die man Urobilinogene nennt, weiter abgebaut wird. Die Urobilinogene werden über den Stuhl und - nach teilweiser Rückresorption aus dem Darm - auch über die Niere ausgeschieden.

Erhöhungen des Bilirubinspiegels treten bei übermäßigem Abbau von Erythrocyten, Leberfunktionsstörungen oder Galleabflußstörungen aus der Leber in den Darm auf. Übersteigt die Bilirubinbildung die Glucuronidierung und anschließende Ausscheidung in die Galle, so kommt es zur Hyperbilirubinämie, wobei das nichtkonjugierte (d.h. an Albumin gebundene) Bilirubin im Plasma erhöht ist. Bilirubin sammelt sich in den Phospholipidmembranen der Zellen an und kann bei genügend hohen Konzentrationen so die Blut-Hirn Schranke überwinden (Kernikterus). Ab einer Konzentration von 20 mg/dl Bilirubin im Serum liegt ein erhöhtes Risiko für neurotoxische Effekte vor. Der Normalbereich der Konzentration an Bilirubin im Blut bei Erwachsenen und Kindern liegt bei unter 1 mg/dl. Bei Patienten mit Crigler-Najjar Syndrom Typ I liegt der Bereich zwischen 20 bis 50 mg/dl.
Durch photochemische Behandlung (Phototherapie) kann Bilirubin in polare, besser wasserlösliche Derivate überführt werden. Bilirubin liegt normalerweise als (Z,Z)-Bilirubin ((4Z,15Z)-Bilirubin) vor. Zwei photochemische Mechanismen zur Umwandlung von Bilirubin sind bei der Phototherapie von Bedeutung:
1.) Konfigurationsänderung zu (4E,15Z)-Bilirubin und zu (4Z,15E)-Bilirubin;
2.) Strukturelle Änderung zu (4E,15Z)- und (4Z,15E)- Cyclobilirubin.

Während bei der Konfigurationsänderung nur instabile Produkte entstehen, die sich wieder in (Z,Z)-Bilirubin umwandeln können, ist Cyclobilirubin stabil. Weitere Reaktionen wie die Photooxidation zu Biliverdin und die Photoaddition an Albumin-gebundenes Bilirubin treten nur in sehr geringem Maße auf (vergleiche S. Yasuda et al., Pediatr. Int. 43: 270 - 275, 2001).

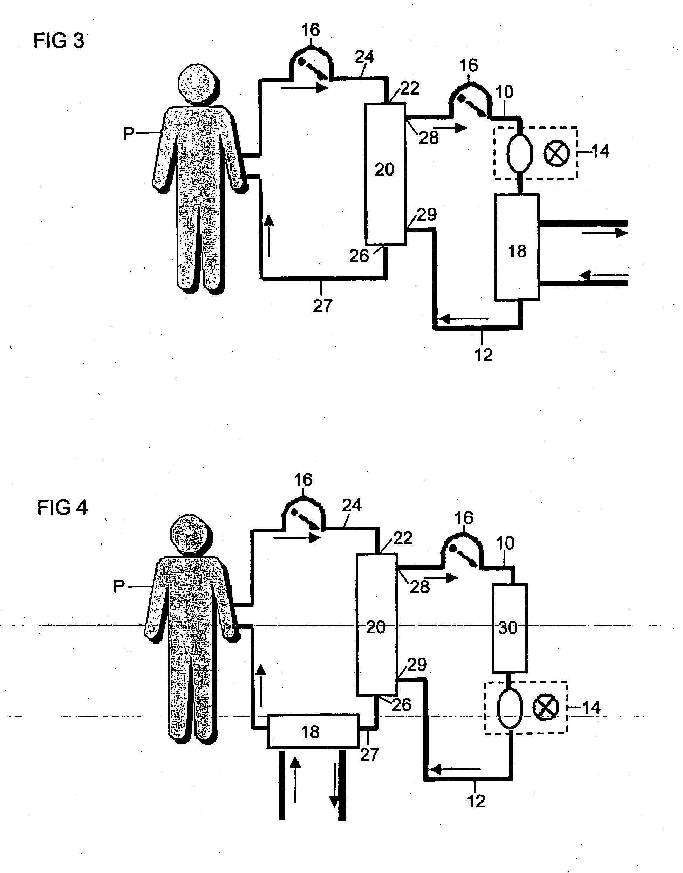

Das Verhältnis von (E,Z)-Bilirubin zu (Z,Z)-Bilirubin nimmt mit steigender Lichtintensität zu, bis es bei etwa 3 µW/(cm² nm) ein Plateau (Photoequilibrium) bei einem Verhältnis von ca. 0,3 erreicht. Die Reaktion zu Cyclobilirubin steigt linear mit steigender Lichtintensität an, ohne ein Photoequilibrium zu erreichen (vergleiche S. Yasuda et al., Pediatr. Int. 43: 270 - 275, 2001, Fig. 3 und Fig. 4). Die Photoisomerisation tritt bei Einstrahlung mit Licht im Wellenlängenbereich von 450 bis 530 nm auf.

(Z,Z)-Bilirubin ist praktisch nicht wasserlöslich, da es intramolekular Wasserstoffbrücken ausbildet. Im Gegensatz dazu sind die Photoisomere wasserlöslich, da an mindestens einer Stelle die intramolekularen Wasserstoffbrücken aufgebrochen wurden, welche für das (Z,Z)-Bilirubin charakteristisch sind. Cyclobilirubin ist deshalb sehr polar und besser wasserlöslich als (Z,Z)-Bilirubin. Die Photoisomere lassen sich mit Wasser aus Chloroform extrahieren, wobei die Extraktion mit steigendem pH besser wird (vergleiche P. Manitto et al., Pediatr. Res. 18: 378 - 381, 1984). Die Photoisomere können in unkonjugierter Form über die Galle/Darm und Niere ausgeschieden werden. Die bei Phototherapie im Urin gefundene Konzentration an Cyclobilirubin liegt bei 0,5 bis 2,5 mg/dl, in der Gallenflüssigkeit ist die Konzentration doppelt so hoch. Die Konzentration an nativem (Z,Z)-Bilirubin im Urin ist gegenüber der Normalkonzentration praktisch nicht erhöht (<0.2 mg/dl).

Bei Neugeborenen kommt es relativ häufig zu einer erhöhten Bilirubinkonzentration im Blut (Gelbsucht), da bei der Angleichung des vor der Geburt sehr hohen Hämatokrits viel Hämoglobin abgebaut wird und die Leber noch nicht so leistungsfähig ist. Ein gängiges Verfahren zur Behandlung der Gelbsucht bei Neugeborenen ist die Bestrahlung der Haut mit blauem bis grünem Licht. Dabei wird Bilirubin durch Photoisomeriation in eine wasserlösliche Form überführt, welche über Darm und Niere eliminiert werden kann. Je nach Alter liegt bei Neugeborenen der Grenzwert bei der Phototherapie initiiert werden soll zwischen 15 und 20 mg/dl Bilirubin im Serum.

Phototherapie wird über das Neugeborenenstadium hinaus bei Patienten mit Crigler-Najjar-Syndrom eingesetzt. Diese haben einen angeborenen Defekt des Enzyms UDP-Glucuronyltransferase, welches Bilirubin in der Leber durch Konjugation mit Glucuronsäure in eine wasserlösliche Form überführt. Die Phototherpie wird mit dem Beginn der Pubertät jedoch zunehmend ineffektiv. Die Zunahme der Hautdicke und der Hautpigmentierung sowie das ungünstiger werdende Verhältnis von Körperoberfläche zu Volumen verhindert die Anwendung der Phototherapie bei erwachsenen Patienten (vergleiche J.R. Chowdhury et al., Hepatology, verlegt durch D. Zakim, TD Boyer Saunders 2002)

Der vorliegenden Erfindung liegt somit die Aufgabe zugrunde, eine neue Vorrichtung zur extrakorporalen Bestrahlung einer Bilirubin enthaltenden Flüssigkeit bereitzustellen, wobei Bilirubin in seine Photoisomere überführt wird. Ferner ist es eine Aufgabe der Erfindung, ein entsprechendes Verfahren anzugeben.

JP 63275351 offenbart eine Vorrichtung zur extrakorporalen Bestrahlung von Bilirubin enthaltendem Blutplasma eines Patienten, welches durch Licht im Wellenlängenbereich 400-500nm bestrahlt wird und woraus anschließend die optischen Isomere von Bilirubin mittels einem Adsorber entfernt werden.

Diese Aufgabe wird erfindungsgemäß durch die Vorrichtung nach Anspruch 1 gelöst. Bevorzugte Ausführungsformen sind Gegenstand der abhängigen Ansprüche.

Erfindungsgemäß umfaßt die Vorrichtung eine erste und eine zweite mit dem Patienten verbindbare Leitungseinrichtung, eine zwischen der ersten und der zweiten Leitungseinrichtung angeordnete Körperflüssigkeitsbestrahlungseinrichtung und zumindest eine steuerbare, in der ersten und/oder der zweiten Leitungseinrichtung angeordnete Körperflüssigkeitsfördereinrichtung. Durch die Körperflüssigkeitsfördereinrichtung ist ein steuerbarer Durchfluß der Körperflüssigkeit in der Vorrichtung erzeugbar, wobei die erste Leitungseinrichtung dafür ausgelegt ist, die dem Patienten entnommene und zu bestrahlende Körperflüssigkeit der Körperflüssigkeitsbestrahlungseinrichtung kontinuierlich zuzuführen und die zweite Leitungseinrichtung dafür ausgelegt ist, die bestrahlte Körperflüssigkeit dem Patienten kontinuierlich zuzuführen.

Eine mit dem Patienten verbindbare Leitungseinrichtung kann direkt mit dem Patienten verbunden sein. Alternativ können optional weitere Leitungseinrichtungen vorgesehen sein über welche der Patient mit der ersten bzw. zweiten Leitungseinrichtung in Flüssigkeitsverbindung steht.

Die Körperflüssigkeitsbestrahlungseinrichtung bzw. Flüssigkeitsbestrahlungseinrichtung weist vorzugsweise einen zumindest teilweise lichtdurchlässigen Durchflußbehälter und eine dem Durchflußbehälter vorzugsweise benachbarte Strahlungsquelle auf. Die Strahlungsquelle emittiert elektromagnetische Strahlung mit Wellenlängen größer als 430 nm, besonders bevorzugt in einem Wellenlängenbereich von 450 bis 530 nm. Die Strahlungsintensität ist bevorzugt größer als 4 µW/(cm² nm). Die Strahlungsintensität der Phototherapie wird häufig als Strahlungsdichte pro Bandbreite angegeben. Dazu wird die gemessene Strahlungsdichte durch die spektrale Bandbreite des Sensors geteilt. Die Bandbreite von Phototherapiesensoren ist dem Spektralbereich angepasst, in dem die Photoreaktion auftritt, d.h. in der vorliegenden Erfindung zwischen 430 und 530 nm. Strahlungsdosen von weniger als 4 µW/(cm² nm) sind nicht effektiv. Der zumindest teilweise lichtdurchlässige Durchflußbehälter kann z.B. eine Durchflußküvette sein. Die Mindestanforderungen an den Durchflußbehälter sind eine gute Lichtdurchlässigkeit im Wellenlängenbereich von 400 bis 530 nm und ein so großes Volumen, daß die Flüssigkeit eine gewisse Verweilzeit in der Küvette hat, damit die photochemische Umsetzung stattfinden kann.

Die Körperflüssigkeitsfördereinrichtung bzw. Flüssigkeitsfördereinrichtung ist hinsichtlich ihrer Flüssigkeitsförderrate steuerbar und kann somit einen regulierbaren Durchfluß der Körperflüssigkeit bzw. Flüssigkeit durch die Vorrichtung erzeugen. Die Flüssigkeitsförderrate liegt in einem Bereich von 100 bis 500 ml/min. Bei der Lebereersatztherapie liegt sie bevorzugt bei 300 ml/min. Die Körperflüssigkeitsfördereinrichtung kann z.B. eine Pumpe sein.

Durch die oben genannte Vorrichtung wird das Bilirubin zwar in seine wasserlöslichen Photoisomere überführt, diese werden jedoch nicht durch die Vorrichtung eliminiert. Dies setzt vorraus, daß der Patient noch eine ausreichende Nierenfunktion aufweist, damit die Photoisomere, ähnlich wie bei der Behandlung Neugeborener, über die Niere ausgescheiden werden können.

Deshalb weist die erfindungsgemäße Vorrichtung in der zweiten Leitungseinrichtung eine Photoisomer-Entfernungseinrichtung zum Entfernen der Photoisomerisationsprodukte von Bilirubin auf. Somit kann die bestrahlte Körperflüssigkeit ohne die Photoisomerisationsprodukte von Bilirubin dem Patienten zugeführt werden. Diese Vorrichtung ist als Dialysator ausgebildet. Vorzugsweise kann mit der Hilfe von Adsobern die Effektivität der Bilirubinentfernung deutlich gesteigert werden, da die Bindungskonstanten von (Z,E)-Bilirubin und von Cyclobilirubin an Albumin deutlich kleiner sind als die Bindungskonstante von Bilirubin an Albumin.

In einer weiteren bevorzugten Ausführungsform weist die Vorrichtung einen Filter, insbesondere einen Plasmafilter, mit einer ungefilterten und einer gefilterten Seite auf. Die ungefilterte Seite wird von der gefilterten Seite durch zumindest ein Filtermaterial, beispielsweise eine Filtermembran, getrennt, wobei ein Flüssigkeitszuführeingang der ungefilterten Seite mit einer dritten mit dem Patienten verbindbaren Leitungseinrichtung verbunden ist, ein Flüssigkeitsabführausgang der ungestörten Seite mit einer vierten mit dem Patienten verbindbaren Leitungseinrichtung verbunden ist , ein Flüssigkeitsabführausgang der gefilterten Seite mit der ersten Leitungseinrichtung verbunden ist und ein Flüssigkeitszuführeingang der gefilterten Seite mit der zweiten Leitungseinrichtung verbunden ist. Somit ist die erste Leitungseinrichtung über die dritte Leitungseinrichtung und die zweite Leitungseinrichtung über die vierte Leitungseinrichtung mit dem Patienten verbindbar. Der Plasmafilter weist vorzugsweise einen Druckgradienten auf, so daß im vorderen Bereich des Filters filtriert wird (Flüssigkeitsübertritt von der ungefilterten zur gefilterten Seite) und im hinteren Bereich des Filters rückfiltiert wird (Flüssigkeitsübertritt von der gefilterten auf die ungefilterte Seite), d.h. die bestrahlte Flüssigkeit wird dem Patienten wieder zugeführt.

Der Filter kann die zellulären Bestandteile des Blutes zurückhalten, wobei Albumin mit dem daran gebundenen Bilirubin sowie weitere kleinmolekulare Substanzen aber durchgelassen werden. Somit kann der Albumin-Bilirubin-Komplex in die erste Leitungseinrichtung gelangen, in der Körperflüssigkeitsbestrahlungseinrichtung bestrahlt werden und die resultierenden Photoisomere anschließend über die zweite bzw. vierte Leitungseinrichtung dem Patienten zugeführt werden. Die Bestrahlung kann auf der Seite des plasmahaltigen Filtrats als auch auf der Seite der angereicherten Flüssigkeit erfolgen. Eine Bestrahlung auf der Seite des plasmahaltigen Filtrats bietet den Vorteil, daß das Licht nicht wie auf der anderen Seite durch die Absorption von Hämoglobin geschwächt wird. Alternativ zum Plasmafilter kann ein Zellseparator zur Trennung von Blut in Plasma und zelluläre Bestandteile eingebaut sein. Dieser Zellseparator kann beispielsweise eine Zentrifuge sein.

Die erfindungsgemäße Vorrichtung kann ebenfalls vor oder nach der Körperflüssigkeitsbestrahlungseinrichtung einen Adsorber aufweisen. Dieser Adsorber kann beispielsweise ein Anionenaustauscher oder ein unspezifischer Adsorber sein. Während der Anionenaustauscher Bilirubin, Gallensäuren und andere Ionen bindet, kann der unspezifische Adsorber, beispielsweise ein Neutralharz, andere Albumin-gebundene Toxine zurückhalten.

Eine weitere erfindungsgemäße Vorrichtung ist ein Leitungssystem, welches eine erste und eine zweite mit dem Patienten verbindbare Leitungseinrichtung und einen zwischen der ersten und zweiten Leitungseinrichtung angeordneten und damit flüssigkeitsdicht verbundenen Durchflußbehälter umfaßt. Der Durchflußbehälter ist für elektromagnetische Strahlung mit Wellenlängen größer als 430 nm, vorzugsweise in einem Wellenlängenbereich von 450 bis 530 nm, zumindest teilweise lichtdurchlässig. Außerdem weist er vorzugsweise ein so großes Volumen auf, daß die Flüssigkeit eine gewisse Verweilzeit im Durchflußbehälter hat, damit eine photochemische Umsetzung des Bilirubins stattfinden kann. Dieses Leitungssystem kann vorzugsweise als Disposable für die erfindungsgemäße Vorrichtung verwendet werden.

Die erfindungsgemäße Vorrichtung kann vorzugsweise bei der Behandlung von Patienten mit Leberversagen eingesetzt werden, bei denen noch eine ausreichende Nierenfunktion vorhanden ist. Über die Niere können dann die Photoisomere von Bilirubin, ähnlich wie bei der Behandlung Neugeborener, ausgeschieden werden. Eine Kombination mit bioartifiziellen Lebersystemen wäre auch vorteilhaft. Die Aufgabe des Enzyms UDP-Glucuronyltransferase würde dann weitgehend von der Flüssigkeitsbestrahlungseinrichtung übernommen werden.

Des weiteren wird ein Verfahren zur Reduzierung des Bilirubinspiegels in einem Patienten offenbart, worin eine dem Patienten entnommene, Bilirubin enthaltende Körperflüssigkeit einer extrakorporalen Bestrahlung unterzogen wird, wobei das in der Körperflüssigkeit enthaltene Bilirubin in Photoisomerisationsprodukte überführt wird, deren Wasserlöslichkeit höher als Bilirubin ist und die bestrahlte Körperflüssigkeit, welche die derart erzeugten Photoisomeriationsprodukte enthält, in den Patienten rückgeführt wird.

Das Verfahren kann vorzugsweise mittels der vorher beschriebenen Ausführungsformen erfindungsgemäßer Vorrichtungen durchgeführt werden.

Das Verfahren ist vorzugsweise zur Behandlung erhöhter Bilirubinspiegel bei allen Formen von Leberversagen vorgesehen. Das erfindungsgemäße Verfahren ist einfach anzuwenden, kostengünstig und unkritisch in Bezug auf die Sicherheit der Anwendung. Das Verfahren läßt sich einfach mit jedem bestehenden Verfahren zur Leberersatztheraphie, wie die Albumindialyse oder Bilirubinadsorber, kombinieren. Da die Photoisomere eine kleinere Bindingskonstante zu Albumin haben als Bilirubin ist somit beispielsweise eine deutliche Erhöhung der Effektivität der Bilirubinentfernung möglich.

Die Erfindung wird im folgenden mit Bezug auf begleitende Zeichnungen beispielhaft beschrieben. Es zeigt:
- Fig. 1: ein schematisches Blockschaltbild einer Vorrichtung, welche nicht Gegenstand der Erfindung ist, mit erster Leitungseinrichtung 10, zweiter Leitungseinrichtung 12, Körperflüssigkeitsbestrahlungseinrichtung 14 und Körperflüssigkeitsfödereinrichtung 16;
- Fig. 2: ein schematisches Blockschaltbild einer bevorzugten Ausführungsform einer erfindungsgemäßen Vorrichtung mit erster Leitungseinrichtung 10, zweiter Leitungseinrichtung 12, Körperflüssigkeitsbestrahlungseinrichtung 14, Körperflüssigkeitsfödereinrichtung 16 und Photoisomer-Entfernungseinrichtung 18;
- Fig. 3: ein schematisches Blockschaltbild einer weiteren bevorzugten Ausführungsform einer erfindungsgemäßen Vorrichtung mit erster Leitungseinrichtung 10, zweiter Leitungseinrichtung 12, Flüssigkeitsbestrahlungseinrichtung 14, Flüssigkeitsfödereinrichtung 16, Photoisomer-Entfernungseinrichtung 18, Filter/Zellseparator 20, dritter Leitungseinrichtung 24, vierter Leitungseinrichtung 27, Flüssigkeitszuführeingang 22 der ungefilterten Seite Flüssigkeitsabführausgang 26 der ungefilterten Seite Flüssigkeitsabführausgang 28 der gefilterten Seite und Flüssigkeitszuführeingang 29 der gefilterten Seite;
- Fig. 4: ein schematisches Blockschaltbild einer weiteren bevorzugten Ausführungsform einer erfindungsgemäßen Vorrichtung mit erster Leitungseinrichtung 10, zweiter Leitungseinrichtung 12, Flüssigkeitsbestrahlungseinrichtung 14, Flüssigkeitsfödereinrichtung 16, Photoisomer-Entfernungseinrichtung 18, Filter 20, dritter Leitungseinrichtung 24, vierter Leitungseinrichtung 27, Flüssigkeitszuführeingang 22 der ungefilterten Seite Flüssigkeitsabführausgang 26 der ungefilterten Seite Flüssigkeitsabführausgang 28 der gefilterten Seite, Flüssigkeitszuführeingang 29 der gefilterten Seite und Adsober 30;

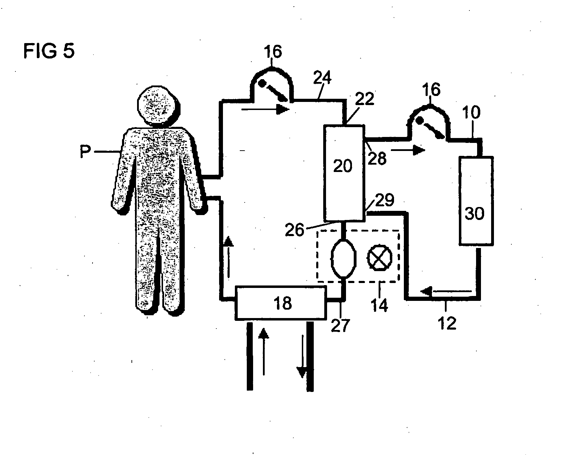

- Fig. 5: ein schematisches Blockschaltbild einer weiteren bevorzugten Ausführungsform einer erfindungsgemäßen Vorrichtung mit erster Leitungseinrichtung 10, zweiter Leitungseinrichtung 12, Flüssigkeitsbestrahlungseinrichtung 14, Flüssigkeitsfödereinrichtung 16, Photoisomer-Entfernungseinrichtung 18, Filter 20, dritter Leitungseinrichtung 24, vierter Leitungseinrichtung 27, Flüssigkeitszuführeingang 22 der ungefilterten Seite Flüssigkeitsabführausgang 26 der ungefilterten Seite Flüssigkeitsabführausgang 28 der gefilterten Seite, Flüssigkeitszuführeingang 29 der gefilterten Seite und Adsober 30;
- Fig. 6: die Photoisomeriation von (4Z,15Z)-Bilirubin zu (4E,15Z)-Bilirubin und (4Z,15E)-Bilirubin;
- Fig.7: die Photoisomerisation von (4Z,15E)-Bilirubin zu Cyclobilirubin;
- Fig. 8: ein schematisches Bild des experimentellen Versuchsaufbaus zur Bilirubin Phototherapie im Hämofiltrationsmodus (Beispiel 1);
- Fig.9: den Verlauf der Konzentration an Cyclobilirubin im Filtrat in Beispiel 1 bei Bestrahlung.

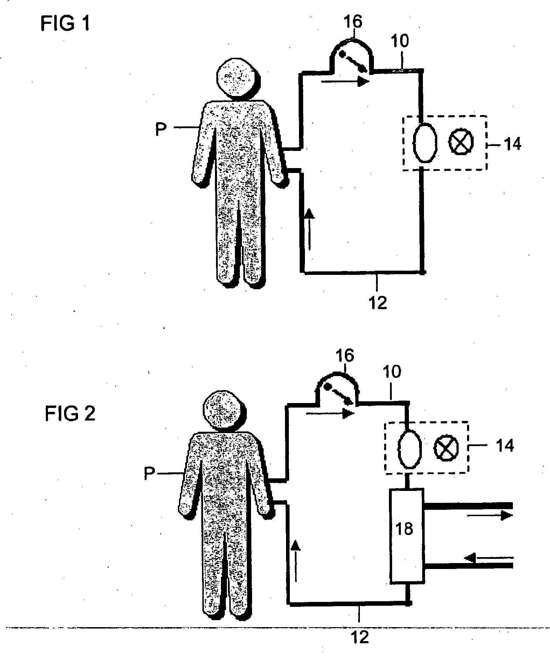

Die in **Fig. 1** gezeigte Vorrichtung, welche nicht Gegenstand der Erfindung ist, zeigt eine erste Leitungseinrichtung 10 mit einem ersten und einem zweiten Ende. Dabei ist das erste Ende der Leitungseinrichtung 10 mit dem Patienten P verbindbar und das zweite Ende mit der Körperfüssigkeitsbestrahlungseinrichtung 14 verbunden. Die zweite Leitungseinrichtung 12 weist ebenfalls ein erstes und ein zweites Ende auf, wobei das erste Ende mit der Körperflüssigkeitsbestrahlungseinrichtung 14 verbunden und das zweite Ende mit dem Patienten P verbindbar ist. Die Durchflußrate, d.h. das geförderte Flüssigkeitsvolumen pro Zeiteinheit, durch diese Leitungseinrichtungen 10, 12 ist über eine Körperflüssigkeitsfördereinrichtung 16 steuerbar. Dadurch kann dem Patienten P über die erste Leitungseinrichtung 10 Körperflüssigkeit, beispielsweise Blut, kontinuierlich entnommen werden, welche dann in der Körperflüssigkeitsbestrahlungeinheit 14 bestrahlt wird. Durch die zweite Leitungseinrichtung 12 wird die bestrahlte Körperflüssigkeit dem Patienten P wieder kontinuierlich zugeführt. Diese Vorrichtung hat den Vorteil, daß keine Fremdsubstanzen, wie beispielsweise Adsorbermaterial, zur Entfernung des Bilirubins auf das Patientenblut/-plasma einwirken. Dadurch ist die Biokompatibilität des Verfahrens sehr hoch und beispielsweise die Gefahr einer Komplementaktivierung erheblich verringert. Dadurch, daß die Vorrichtung direkt mit dem Patienten verbunden werden kann, wird eine Verkürzung der Behandlungszeit und eine Vereinfachung des Verfahrens (beispielsweise Entnahme des Blutes, Bestrahlen usw.) bewirkt, wobei eine sehr schnelle Entfernung des Bilirubins aus dem Blutkreislauf des Patienten ermöglicht wird. Allerdings ist für diese Vorrichtung eine ausreichende Nierenfunktion und möglicherweise eine Restfunktion der Leber beim Patienten notwendig. Diese Vorrichtung ist aber für Patienten mit Crigler-Najjar-Syndrom, bei denen aufgrund des Alters die konventionellen Phototherapie limitiert ist, ideal.

**Fig. 2** zeigt eine zu Fig. 1 ähnlich aufgebaute Vorrichtung, welche zusätzlich nach der Körperflüssigkeitsbestrahlungseinheit 14 eine Photoisomer-Entfernungseinrichtung 18 in der zweiten Leitungseinrichtung 12 aufweist. Diese Photoisomer-Enfernungeinrichtung ist als Dialysator ausgebildet, deshalb, weist sie zusätzlich Zuführ- und Abführleitungen, wie beispielsweise Dialysatleitungen oder eine Substitutionsflüssigkeits-Zuführung, auf. Eine derartige Ausgestaltung ist insbesondere bei Patienten P mit unzureichender Nierenfunktion von Vorteil, da die Photoisomere in der Photoisomer-Entfernungeinrichtung vorzugsweise adsorbiert werden und nicht über die Niere ausgeschieden werden müssen.

**Fig. 3** zeigt eine weitere Ausführungsform der Vorrichtung, wobei die mit der Ausführungsform in Fig. 1 identischen Teile mit identischen Bezugsziffern bezeichnet werden. Die dritte Leitungseinrichtung 24 weist ein erstes und ein zweites Ende auf, wobei das erste Ende mit dem Patienten P verbindbar ist und das zweite Ende mit dem Flüssigkeitszuführeingang 22 einer ungefilterten Seite eines Filters 20 verbunden ist. Der Filter 20 weist neben der ungefilterten Seite eine gefilterte Seite auf, wobei die ungefilterte Seite von der gefilterten Seite durch zumindest ein Filtermaterial getrennt ist. Die vierte Leitungseinrichtung 27 weist ein erstes und ein zweites Ende auf, wobei das erste Ende mit dem Flüssigkeitsabführausgang 26 einer ungefilterten Seite eines Filters 20 verbunden ist und das zweite Ende mit dem Patienten P verbindbar ist. Die Körperflüssigkeit, welche durch den Flüssigkeitszuführeingang 22 der ungefilterten Seite eintritt, kann teilweise durch den Flüssigkeitsabführausgang 28 der gefilterten Seite wieder austreten. Über die erste Leitungseinrichtung 10 wird dann die gefilterte Körperflüssigkeit der Körperflüssigkeitsbestrahlungseinrichtung 14 zugeführt und kann anschließend in der zweiten Leitungseinrichtung 12 optional durch eine Photoisomer-Entfernungseinrichtung 18 fließen. Die gefilterte, bestrahlte Körperflüssigkeit tritt am Flüssigkeitszuführeingang 29 der gefilterten Seite wieder in den Filter 20 ein und durch den Flüssigkeitsabführausgang 26 der ungefilterten Seite in die vierte Leitungseinrichtung aus. In der vierten Leitungseinrichtung 27 treffen somit die ungefilterte Körperflüssigkeit und die gefilterte, bestrahlte und optional photosiomerfreie Körperflüssigkeit wieder aufeinander. Wenn es sich bei der Körperflüssigkeit beispielsweise um Blut handelt, wird auf diese Weise nur das Blutplasma bestrahlt, da durch den Filter 20 alle zellulären Bestandteile des Blutes abgetrennt werden. Alternativ zum Filter 20 kann ein Zellseparator, beispielsweise eine Zentrifuge, zur Trennung von Blut in Plasma und zelluläre Bestandteile verwendet werden.

Vorteil dieser erfindungsgemäßen Vorrichtung ist im Gegensatz zur beispielsweise Vollblutbestrahlung die höhere Effektivität der Bestrahlung. Da Hämoglobin auch Lichtadsorption in diesem Wellenlängenbereich zeigt, können so unerwünschte Nebenreaktionen, durch die es beispielsweise bei hohen Lichtintensitäten kommen würde, beispielsweise Erwärmung oder Photoreaktionen, ausgeschlossen werden.

Durch zumindest eine steuerbare Flüssigkeitsfödereinrichtung 16 in der ersten 10 und/oder der zweiten 12 Leitungseinrichtung und zumindest eine steuerbare Körperflüssigkeitsfördereinrichtung in der dritten 24 und/oder der vierten 27 Leitungseinrichtung kann die Flußrate der Flüssigkeit auf der gefilterten Seite optional wesentlich größer sein als auf der ungefilterten Seite, d.h. auf der gefilterten Seite herrscht Rezirkulation. Dadurch wird die Photoisomer-Entfernungseinrichtung besser genutzt, da die gefilterte Flüssigkeit mehrfach über sie hinwegfließt.

Wenn der Filter 20 ein Dialysator ist, befindet sich auf der Permeatseite des Dialysators eine Albuminlösung. Das Bilirubin aus der Körperflüssigkeit diffundiert dann ohne Albumin auf die Permeatseite des Dialysators. Dort wird es durch die Bestrahlung in seine Photoisomere überführt und durch die Photoisomer-Entfernungseinrichtung 18 entfernt, d.h. die Photoisomere gelangen nicht in den Patienten P zurück. Da aber die Bindungskonstanten des (Z,E)-Bilirubins und des Cyclobilirubins an Albumin deutlich kleiner sind als die Bindungskonstante von (Z,Z)-Bilirubin an Albumin ist die Entfernung von Bilirubin mit Hilfe der Photoisomer-Entfernungseinrichtung 18, einem Dialysator, deutlich effektiver.

**Fig. 4** zeigt eine weitere bevorzugte Ausführungsform, welche ähnlich zu derjenigen von Fig. 3 aufgebaut ist. Bei der Ausführungsform gemäß Fig. 4 ist vor der Körperflüssigkeitsbestrahlungseinrichtung 14 noch ein Adsorber 30 in der ersten Leitungseinrichtung 10 angeordnet und die optionale Photoisomer-Entfernungseinrichtung 18 ist erst nach der Zusammenführung von ungefilterter Körperflüssigkeit und gefilterter, bestrahlter Körperflüssigkeit in der vierten Leitungseinrichtung 27 angeordnet. Durch die Verbindung der erfindungsgemäßen Vorrichtung mit einem Adsorber können beispielsweise andere Albumin-Toxine in der Vorrichtung gebunden werden, so daß in einem Schritt nicht nur Bilirubin aus dem Blut entfernt werden kann sondern auch unerwünschte Toxine.

**Fig. 5** zeigt eine weitere bevorzugte Ausführungsform, welche ähnlich zu derjenigen von Fig. 3 aufgebaut ist. Bei der Ausführungsform gemäß Fig. 5 ist die Körperflüssigkeitsbestrahlungseinrichtung 14 in der vierten Leitungseinrichtung 27 nach dem Zusammenführen von ungefilterter Körperflüssigkeit und gefilterter, mit dem Adsorber behandelter, Flüssigkeit angeordnet und die optionale Photoisomer-Entfernungseinrichtung 18 ist in der vierten Leitungseinrichtung 27 nach der Körperflüssigkeitsbestrahlungseinrichtung 14 angeordnet.

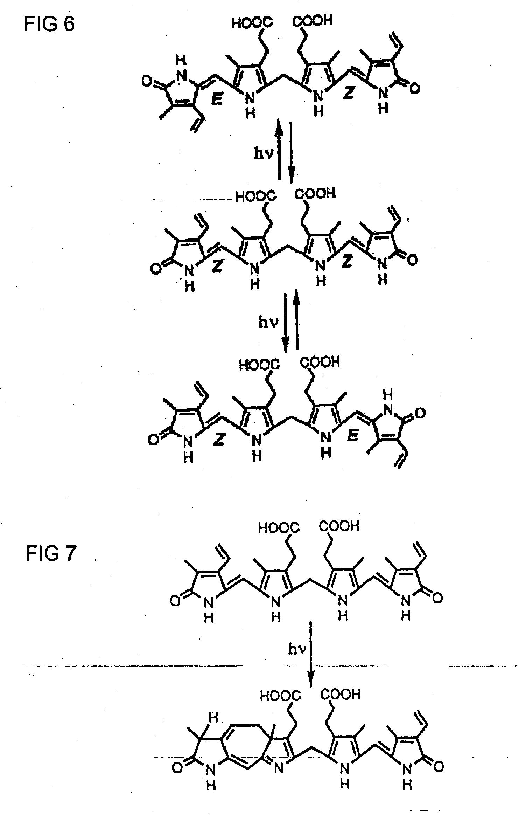

**Fig. 6** zeigt die im Stand der Technik bekannte Photoisomerisation von (4Z,15Z)-Bilirubin zu (4E,15Z)-Bilirubin und (4Z,15E)-Bilirubin.

**Fig. 7** zeigt die im Stand der Technik bekannte Photoisomerisation von (4Z,15E)-Bilirubin zu Cyclobilirubin.

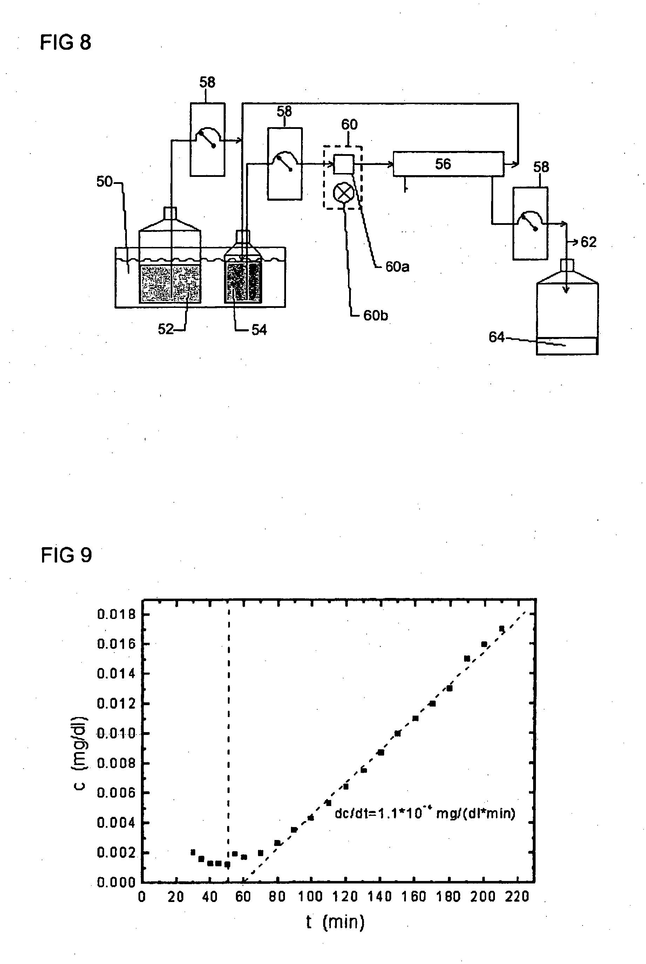

**Fig. 8** zeigt einen in-vitro Versuchsaufbau einer erfindungsgemäßen Vorrichtung mit einem Wasserbad 50, welches vorzugsweise bei 37 °C gehalten wird. In diesem Wasserbad 50 befinden sich eine Bilirubin/HSA-Lösung 54 und eine Pufferlösung 52. Durch die Pumpe 58 wird ein kontinuierlicher Durchfluß durch die Leitungseinrichtungen, die Flüssigkeitsbestrahlungseinrichtung 60 und den Hämofilter 56 erzeugt. Die Fließrichtung der Flüssigkeit ist durch die Pfeile gekennzeichnet. Die Flüssigkeitsbestrahlungseinrichtung besteht aus einer Strahlungsquelle 60b und einer arteriellen Blasenkammer als Küvette 60a. Das Volumen der Bilirubin/HSA-Lösung bleibt während der gesamten Versuchsdauer annähernd konstant, da das Flüssigkeitsvolumen, welches aus dem in Flussrichtung hinteren Teil des Hämofilters 56 mit einer Pumpe 58 abgepumpt wird, durch eine Pufferlösung 52 über eine Pumpe 58 ersetzt wird. Das aus dem Hämofilter 56 abgepumpte Filtrat 64 kann dann vermessen werden.

Abschließend wird angemerkt, daß obwohl die Erfindung anhand obiger Ausführungsformen beispielhaft beschrieben wurde, einzelne in diesem Zusammenhang offenbarte Merkmale und Elemente auch bei anderen Ausführungsformen Verwendung finden können und somit zu neuen Ausführungsformen zusammengefaßt werden können.

### Beispiel 1:

250 ml einer Bilirubin/HSA-Lösung (Bilirubin: Sigma, Prod.Nr. B4126; HSA: Humanserumalbumin, Biotest Pharma GmbH, Dreieich) mit einer Bilirubinkonzentration von 15 mg/100 ml und einer Albuminkonzentration von 30 g/l wurden wie in Figur 8 dargestellt mit einem Fluss von 100 ml/min durch eine arterielle Blasenkammer (Schlauchset FA104, Fresenius) als Teil der Flüssigkeitsbestrahlungseinrichtung und einen in Reihe geschalteten Hämofilter (F3, Fresenius) rezirkuliert. Zusätzlich wurde aus dem in Flussrichtung hinteren Filtratanschluss des Hämofilters Filtrat abgepumpt. Damit das Volumen der Bilirubin/HSA-Lösung über die gesamte Versuchsdauer annähernd konstant blieb, wurde auf der venösen Seite des Bilirubin/HSA-Kreislaufs mit dem gleichen Fluß, mit der das Filtrat abgepresst wurde, Hydogencarbonat-Pufferlösung (hergestellt mit den Konzentrationen BC-F 8,4% und SK-F 003) substituiert.

Die ersten 50 Minuten des Versuchs erfolgten ohne Bestrahlung, um die wasserlöslichen, nicht-physiologischen Isomere IIIα und XIIIα des Bilirubins, welche in dem Produkt von Sigma zu 6% enthalten sind, zu entfernen. Die Bestrahlung erfolgte anschließend für 160 Minuten mit einer Kaltlichtquelle (KL 1500, Schott) mit vorgesetztem Langpassgilter, der Licht oberhalb von 435 nm durchlässt (Sperrfilter GG 435, AHF Analysentechnik, Tübingen). Die Strahlungsdichte betrug E₄₁₀₋₄₈₅ = 5,25 mW/cm² (70 µW/(cm² nm). Die Konzentration von Cyclobilirubin im Filtrat wurde im Spektralphotometer bei einer Wellenlänge von 440 nm in einer Küvette von 10 cm Schichtdicke gemessen.

Fig. 9 zeigt den Verlauf der Konzentration von Cyclobilirubin im Filtrat während des Hämofiltrationsversuchs. Die gemessene Extinktion wurde dabei mit dem Literaturwert des Extinktionskoeffizienten von Cyclobilirubin (e=33000M⁻¹cm⁻¹) in die Konzentration umgerechnet.

Das Experiment zeigt während des Ablaufs im Dunkeln während der ersten 50 Minuten das Herrausspülen der nicht-physiologischen Isomere des Bilirubins. Nach Einschalten der Strahlungsquelle zeigt sich ein deutlicher Anstieg der Konzentration von 1,1 x 10⁻⁴ mg/100 ml pro Minute. Die Konzentration im Filter entspricht der Konzentration an freien, d.h. nicht an Albumin gebundenen, Photoisomeren in der Bilirubin/HSA-Lösung nach der Bestrahlung.

Das Experiment zeigt, das durch Bestrahlung von Albumin-gebundenem Bilirubin freie Photoisomere enstehen, die über einen Hämofilter entfernt werden können. Dadurch ist das Prinzip der extrakorporalen Bilirubin-Phototherapie nachgewiesen.

### Bezugszeichenliste

- 10: erste Leitungseinrichtung
- 12: zweite Leitungseinrichtung
- 14: Körperflüssigkeitsbestrahlungseinrichtung/Flüssigkeitsbestrahlungseinrichtung
- 16: Körperflüssigkeitsfördereinrichtung/Flüssigkeitsfördereinrichtung
- 18: Photoisomer-Entfernungseinrichtung
- 20: Filter/Zellseparator/Dialysator/Hämofilter
- 22: Flüssigkeitszuführeingang des Filters/Dialysators/Hämofilters auf der ungefilterten bzw. zu dialysierenden Seite
- 24: dritte Leitungseinrichtung
- 26: Flüssigkeitsabführausgang des Filters/Dialysators/Hämofilters auf der ungefilterten bzw. zu dialysierenden Seite
- 27: vierte Leitungseinrichtung
- 28: Flüssigkeitsabführausgang des Filters auf der gefilterten bzw. Permeatseite
- 29: Flüssigkeitszuführeingang des Filters/Dialysator/Hämofilter auf der gefilterten bzw. Permeatseite
- 30: Adsorber

## Patentansprüche

1. Vorrichtung zur extrakorporalen Bestrahlung von einer Bilirubin enthaltenden Körperflüssigkeit eines Patienten (P), umfassend eine erste (10) und eine zweite (12) mit dem Patienten (P) verbindbare Leitungseinrichtung, eine zwischen der ersten (10) und der zweiten (12) Leitungseinrichtung angeordnete und damit flüssigkeitsdicht verbundene Körperflüssigkeitsbestrahlungseinrichtung (14) und zumindest eine steuerbare, in der ersten (10) und/oder zweiten (12) Leitungseinrichtung angeordnete Körperflüssigkeitsfördereinrichtung (16), worin mittels der zumindest einen Körperflüssigkeitsfördereinrichtungen (16) ein steuerbarer Durchfluß der Körperflüssigkeit durch die Leitungseinrichtungen (10, 12) und die Körperflüssigkeitsbestrahlungseinrichtung (14) erzeugbar ist, wobei die erste Leitungseinrichtung (10) ausgelegt ist, die dem Patienten (P) entnommene und zu bestrahlende Körperflüssigkeit der Körperflüssigkeitsbestrahlungseinrichtung (14) kontinuierlich zuzuführen und die zweite Leitungseinrichtung (12) ausgelegt ist, die bestrahlte Körperflüssigkeit dem Patienten (P) kontinuierlich zuzuführen und wobei die Körperflüssigkeitsbestrahlungseinrichtung (14) eine Strahlungsquelle zur Emission elektromagnetischer Strahlung mit Wellenlängen größer als 430 nm, vorzugsweise in einem Wellenlängenbereich von 450 bis 530 nm, umfaßt,
wobei in der zweiten Leitungseinrichtung (12) eine Photoisomer-Entfernungseinrichtung (18) zur Entfernung von wasserlöslichen Photoisomeren von Bilirubin angeordnet ist, die als Dialysator ausgebildet ist.

2. Vorrichtung nach Anspruch 1, welche weiterhin einen Filter (20) mit einer ungefilterten Seite und einer gefilterten Seite aufweist, wobei die ungefilterte Seite von der gefilterten Seite durch zumindest ein Filtermaterial getrennt ist, wobei
- ein Flüssigkeitszuführeingang (22) der ungefilterten Seite mit einer dritten (24) mit dem Patienten verbindbaren Leitungseinrichtung verbunden ist,
- ein Flüssigkeitsabführausgang (26) der ungefilterten Seite mit einer vierten (27) mit dem Patienten verbindbaren Leitungseinrichtung verbunden ist,
- ein Flüssigkeitsabführausgang (28) der gefilterten Seite mit der ersten Leitungseinrichtung (10) verbunden ist, und
- ein Flüssigkeitszuführeingang (29) der gefilterten Seite mit der zweiten Leitungseinrichtung (12) verbunden ist.

3. Vorrichtung nach Anspruch 2, wobei in der ersten Leitungseinrichtung (10) vor der Körperflüssigkeitsbestrahlungseinrichtung (14) ein Adsorber (30) angeordnet ist.

4. Vorrichtung nach einem der vorhergehenden Ansprüche, wobei die Körperflüssigkeitsbestrahlungseinrichtung (14) eine Strahlungsquelle und einen zumindest teilweise lichtdurchlässigen Durchflußbehälter umfaßt.

5. Vorrichtung nach einem der vorhergehenden Ansprüche, wobei die Strahlungsintensität der Strahlungsquelle größer als 4 µW/(nm cm²) ist.

6. Vorrichtung nach einem der vorhergehenden Ansprüche, wobei der zumindest teilweise lichtdurchlässige Durchflußbehälter eine Durchflußküvette ist.

7. Vorrichtung nach einem der vorhergehenden Ansprüche, wobei die Körperflüssigkeitsfördereinrichtung (16) eine Pumpe ist.

8. Vorrichtung nach einem der vorhergehenden Ansprüche, wobei die Körperflüssigkeit Blut oder Blutplasma ist.

9. Vorrichtung nach Anspruch 1, weiterhin umfassend eine dritte (24) und vierte (27) Leitungseinrichtung, zumindest eine steuerbare in der dritten (24) und/oder vierten (27) Leitungseinrichtung angeordnete Körperflüssigkeitsfördereinrichtung (16) und einen Dialysator (20), wobei die Permeatseite des Dialysators mit der ersten (10) und der zweiten (12) Leitungseinrichtung verbunden ist und die zu dialysierende Seite des Dialysators an ihrem Dialysatoreingang (22) mit der dritten mit einem Patienten (P) verbindbaren Leitungseinrichtung (24) und an ihrem Dialysatorausgang (26) mit der vierten mit dem Patienten (P) verbindbaren Leitungseinrichtung (27) verbunden ist, worin mittels der Flüssigkeitsfördereinrichtungen (16) ein steuerbarer Durchfluß der Flüssigkeit durch die Leitungseinrichtungen (10, 12, 24, 27) und die Flüssigkeitsbestrahlungseinrichtung (14) erzeugbar ist.

10. Vorrichtung nach Anspruch 9, wobei in der ersten Leitungseinrichtung (10) vor der Flüssigkeitsbestrahlungseinrichtung (14) ein Adsorber (30) angeordnet ist.

11. Vorrichtung nach Anspruch 9 oder 10, wobei die Flüssigkeitsbestrahlungseinrichtung (14) in der vierten Leitungseinrichtung (27) vor der Photoisomer-Entfernungseinrichtung (18) angeordnet ist.

12. Vorrichtung nach einem der Ansprüche 9 bis 11, wobei die Flüssigkeitsbestrahlungseinrichtung (14) eine Strahlungsquelle und einen zumindest teilweise lichtdurchlässigen Durchflußbehälter umfaßt.

13. Vorrichtung nach einem der Ansprüche 9 bis 12, wobei die Strahlungsintensität der Strahlungsquelle größer als 4 µW/(nm cm²) ist.

14. Vorrichtung nach einem der Ansprüche 9 bis 13, wobei der zumindest teilweise lichtdurchlässige Durchflußbehälter eine Durchflußküvette ist.

15. Vorrichtung nach einem der Ansprüche 9 bis 14, wobei die Flüssigkeitsfördereinrichtung (16) eine Pumpe ist.

16. Vorrichtung nach einem der Ansprüche 9 bis 15, wobei die Körperflüssigkeit auf der zu dialysierenden Seite Blut oder Blutplasma und die Flüssigkeit auf der Permeatseite eine Albuminlösung ist.

## Claims

1. Device for extracorporeal irradiation of a patient's (P) bodily fluid containing bilirubin, comprising a first (10) and a second (12) line which can be connected to the patient (P), an irradiation unit for bodily fluids (14) located between the first (10) and the second (12) lines and connected therewith in a fluid-tight manner, and at least one controllable feed unit for bodily fluids (16) located in the first (10) and/or the second (12) line, wherein by means of said at least one feed unit for bodily fluids (16) a controllable flow of the bodily fluid through the lines (10, 12) and the irradiation unit for bodily fluids (14) is achievable, in which the first line (10) is designed to continually feed the bodily fluid, which was drawn from the patient (P) and is to be irradiated, to the irradiation unit for bodily fluids (14), and the second line (12) is designed to continually feed the irradiated bodily fluid to the patient (P), and wherein the irradiation unit for bodily fluids (14) contains a radiation source for the emission of electromagnetic radiation with wavelengths greater than 430 nm, preferably in a wavelength range from 450 to 530 nm, wherein a photoisomer-removal unit (18) for the removal of water-soluble photoisomers of bilirubin is located in the second line (12), said unit being formed as a dialyzer.

2. Device according to claim 1, which has additionally a filter (20) with an unfiltered side and a filtered side, wherein the unfiltered side is separated from the filtered side by at least one filter material, wherein
- a fluid feed inlet (22) of the unfiltered side is connected to a third (24) patient-connectable line,
- a fluid discharge outlet (26) of the unfiltered side is connected to a fourth (27) patient-connectable line,
- a fluid discharge outlet (28) of the filtered side is connected to the first line (10), and
- a fluid feed inlet (29) of the filtered side is connected to the second line (12).

3. Device according to claim 2, wherein an adsorber (30) is located in the first line (10) before the irradiation unit for bodily fluids (14).

4. Device according to any one of the foregoing claims, wherein the irradiation unit for bodily fluids (14) contains a radiation source and an at least partially translucent flow reservoir.

5. Device according to any one of the foregoing claims, wherein the irradiation intensity of the radiation source is greater than 4 µW/(nm cm²).

6. Device according to any one of the foregoing claims, wherein the at least partially translucent flow reservoir is a flow cuvette.

7. Device according to any one of the foregoing claims, wherein the feed unit for bodily fluids (16) is a pump.

8. Device according to any one of the foregoing claims, wherein the bodily fluid is blood or blood plasma.

9. Device according to claim 1, further comprising a third (24) and fourth (27) line, at least one controllable feed unit for bodily fluids (16) located in the third (24) and/or the fourth (27) line, and a dialyzer (20), wherein the permeate side of the dialyzer is connected to the first (10) and the second (12) line and the dialyzing side of the dialyzer is connected, at its dialyzer inlet (22), to the third patient (P)-connectable line (24) and at its dialyzer outlet (26) to the fourth patient (P)-connectable line (27), wherein by means of the feed unit for bodily fluids (16) a controllable flow of fluid through the lines (10, 12, 24, 27) and the irradiation unit for fluids (14) is achievable.

10. Device according to claim 9, wherein an adsorber (30) is located in the first line (10) before the irradiation unit for bodily fluids (14).

11. Device according to claim 9 or 10, wherein the irradiation unit for fluids (14) is located in the fourth line (27) before the photoisomer-removal unit (18).

12. Device according to any one of claims 9 to 11, wherein the irradiation unit for fluids (14) contains a radiation source and an at least partially translucent flow reservoir.

13. Device according to any one of claims 9 to 12, wherein the irradiation intensity of the radiation source is greater than 4 µW/(nm cm²).

14. Device according to one of claims 9 to 13, wherein the at least partially translucent flow reservoir is a flow cuvette.

15. Device according to any one of claims 9 to 14, wherein the feed unit for fluids (16) is a pump.

16. Device according to any one of claims 9 to 15, wherein the bodily fluid on the dialyzing side is blood or blood plasma and the fluid on the permeate side is an albumin solution.

## Revendications

1. Dispositif pour l'irradiation extracorporelle d'un liquide corporel d'un patient (P) contenant de la bilirubine, comprenant un premier (10) et un deuxième (12) systèmes de conduite pouvant être reliés au patient (P), un système d'irradiation de liquide corporel (14) disposé entre les premier (10) et deuxième (12) systèmes de conduite et relié ainsi de façon étanche aux liquides, et au moins un système d'acheminement de liquide corporel (16) pouvant être commandé, disposé dans le premier (10) et/ou le deuxième (12) système de conduite, dans lequel au moyen de l'au moins un système d'acheminement de liquide corporel (16), un passage pouvant être commandé du liquide corporel peut être généré dans les systèmes de conduite (10, 12) et le système d'irradiation de liquide corporel (14), le premier système de conduite (10) étant conçu pour acheminer en continu le liquide corporel prélevé du patient (P) et à irradier, au système d'irradiation de liquide corporel (14) et le deuxième système de conduite (12) étant conçu pour acheminer en continu le liquide corporel irradié au patient (P) et le système d'irradiation de liquide corporel (14) comprenant une source de rayonnement pour l'émission de rayonnement électromagnétique présentant des longueurs d'ondes supérieures à 430 nm, de préférence dans une plage de longueurs d'ondes de 450 à 530 nm, un système d'élimination de photo-isomères (18) étant disposé dans le deuxième système de conduite (12) pour éliminer les photo-isomères hydrosolubles de la bilirubine, qui est conçu comme un dialyseur.

2. Dispositif selon la revendication 1, qui présente en outre un filtre (20) comportant un côté non filtré et un côté filtré, le côté non filtré étant séparé du côté filtré par au moins un matériau de filtrage, dans lequel :
- une entrée d'acheminement de liquide (22) du côté non filtré est reliée à un troisième (24) système de conduite pouvant être relié au patient,
- une sortie d'évacuation de liquide (26) du côté non filtré est reliée à un quatrième (27) système de conduite pouvant être relié au patient,
- une sortie d'évacuation de liquide (28) du côté filtré est reliée au premier système de conduite (10), et
- une entrée d'acheminement de liquide (29) du côté filtré est reliée au deuxième système de conduite (12).

3. Dispositif selon la revendication 2 dans lequel, dans le premier système de conduite (10), est disposé un adsorbeur (30) avant le système d'irradiation de liquide corporel (14).

4. Dispositif selon l'une des revendications précédentes, dans lequel le système d'irradiation de liquide corporel (14) comprend une source de rayonnement et un conteneur de liquide d'écoulement laissant au moins partiellement passer la lumière.

5. Dispositif selon l'une des revendications précédentes, dans lequel l'intensité d'irradiation de la source d'irradiation est supérieure à 4 µW/(nm cm²).

6. Dispositif selon l'une des revendications précédentes, dans lequel le conteneur de liquide d'écoulement laissant au moins partiellement passer la lumière est une cuvette de liquide d'écoulement.

7. Dispositif selon l'une des revendications précédentes, dans lequel le système d'acheminement de liquide corporel (16) est une pompe.

8. Dispositif selon l'une des revendications précédentes, dans lequel le liquide corporel est le sang ou le plasma sanguin.

9. Dispositif selon la revendication 1, comprenant en outre un troisième (24) et un quatrième (27) système de conduite, au moins un système d'acheminement de liquide corporel (16) pouvant être commandé, disposé dans le troisième (24) et/ou le quatrième (27) système de conduite et un dialyseur (20), dans lequel le côté du perméat du dialyseur est relié au premier (10) et au deuxième (12) système de conduite et le côté de dialyse du dialyseur est relié, à son entrée de dialyseur (22), au troisième système de conduite (24) pouvant être relié à un patient (P) et, à sa sortie de dialyseur (26), au quatrième système de conduite (27) pouvant être relié au patient (P), un passage du liquide pouvant être commandé, pouvant être généré au moyen des systèmes d'acheminement de liquide (16) dans les systèmes de conduite (10, 12, 24, 27) et le système d'irradiation de liquide (14).

10. Dispositif selon la revendication 9, dans lequel dans le premier système de conduite (10) est disposé un adsorbeur (30) avant le système d'irradiation de liquide (14).

11. Dispositif selon la revendication 9 ou 10, dans lequel le système d'irradiation de liquide (14) est disposé dans le quatrième système de conduite (27) avant le système d'élimination de photo-isomères (18).

12. Dispositif selon l'une des revendications 9 à 11, dans lequel le système d'irradiation de liquide (14) comprend une source de rayonnement et un conteneur de liquide d'écoulement laissant au moins partiellement passer la lumière.

13. Dispositif selon l'une des revendications 9 à 12, dans lequel l'intensité d'irradiation de la source d'irradiation est supérieure à 4 µW (nm cm²).

14. Dispositif selon l'une des revendications 9 à 13, dans lequel le conteneur de liquide d'écoulement laissant au moins partiellement passer la lumière est une cuvette de liquide d'écoulement.

15. Dispositif selon l'une des revendications 9 à 14, dans lequel le dispositif d'acheminement de liquide (16) est une pompe.

16. Dispositif selon l'une des revendications 9 à 15, dans lequel le liquide corporel du côté de dialyse est du sang ou du plasma sanguin et le liquide du côté du perméat est une solution d'albumine.
